(19) Europäisches Patentamt

European Patent Office

Office européen des brevets

(11) **EP 1 728 853 A1**

(12) **EUROPEAN PATENT APPLICATION**

(43) Date of publication:
06.12.2006 Bulletin 2006/49

(51) Int Cl.:
*C12M 1/04* (2006.01)          *C12M 1/06* (2006.01)
*C12M 1/36* (2006.01)

(21) Application number: 06114858.1

(22) Date of filing: 01.06.2006

(84) Designated Contracting States:
AT BE BG CH CY CZ DE DK EE ES FI FR GB GR
HU IE IS IT LI LT LU LV MC NL PL PT RO SE SI
SK TR
Designated Extension States:
AL BA HR MK YU

(30) Priority: 02.06.2005 US 686730 P
30.05.2006 US 421065

(71) Applicant: L'AIR LIQUIDE, SOCIETE ANONYME
POUR
L'ETUDE ET L'EXPLOITATION DES PROCEDES
GEORGES CLAUDE
75007 Paris (FR)

(72) Inventors:
• Saucedo, Victor
Willowbrook, MO 60527 (US)
• Brahmbhatt, Sudhir
Glencoe, MO 63038-1446 (US)

(74) Representative: Mellul-Bendelac, Sylvie Lisette
L'Air Liquide,
Service Propriété Industrielle,
75, Quai d'Orsay
75321 Paris Cedex 07 (FR)

(54) **Optimization of process variables in oxygen enriched fermentors through real-time process controls**

(57) Methods and systems are provided for controlling the addition of oxygen in fermentations to achieve a desired oxygen consumption and substrate yield in fermentation cell cultures. In one aspect, the invention provides a method for regulating the addition of oxygen ($O_2$) to a fermentor during a fermentation process, comprising measuring real-time dissolved oxygen (DO) in a fermentation broth, measuring real-time $O_2$ concentration in the fermentor exhaust, and providing the real-time DO measurement and real-time $O_2$ measurement to an adaptive controller configured to regulate $O_2$ flow into the fermentor responsive to the real-time DO measurement and real-time $O_2$ measurement.

Fig. 1

**Description**

This application claims the benefit under 35 U.S.C. § 119(e) to provisional application No. 60/686,730, filed June 02, 2005, the entire contents of which are incorporated herein by reference.

*Field of the Invention*

The invention generally relates to the process productivity in fermentors, and more specifically to a system and method for controlling the addition of oxygen in fermentations to achieve an improved oxygen consumption and substrate yield in microorganisms.

*Description of the Related Art*

Biochemical engineering is a branch of chemical engineering which deals with the design and construction of unit processes involving microorganisms. Fermentation is one example of a process involving the bulk growth of microorganisms on a growth medium. Fermentation is one of the most important processes used in biochemical engineering, and the products of fermentations are extensively used in the pharmaceutical, biotechnology, brewing, and water treatment industries. Fermentations are typically conducted in a fermentor, or bioreactor, which may refer to any vessel or system that supports a biologically active environment. Industrial bioreactors can employ a variety of microorganisms, including bacteria and animal cells, ranging in complexity and response to sheer.

The design of a fermentation system is quite a complex engineering task. Under optimum conditions the microorganisms or cells are able to perform their desired function with great efficiency. The bioreactor's environmental conditions, such as gas (i.e., air, oxygen ($O_2$), nitrogen ($N_2$), carbon dioxide ($CO_2$)) flow rates, temperature, pH, dissolved oxygen levels, and/or agitation speed/circulation rate need to be closely monitored and controlled. To this end, most industrial bioreactor manufacturers use vessels, sensors, and controllers as components of fermentation systems.

Optimal oxygen transfer during a fermentation is perhaps the most difficult task to accomplish. Oxygen solubility in water is extremely low (at the parts per million level), and the solubility is even less in presence of solutes such as nutrients and other additions in broths. Oxygen is also only 20.9% by volume in air. Oxygen transfer in bioreactors can be enhanced by agitation in mechanical fermentors. Agitation is also needed to mix nutrients and to keep the fermentation homogeneous. However, there are limits to the speed of agitation, as it can induce high stress in organisms leading to cell death. High agitation speed also results in higher power consumption, increasing product unit costs. The dissolved oxygen (DO) in the growth media is usually measured to help determine the amount of oxidant gas that should be added to the fermentor.

Many different fermentation systems and their control of oxygen have been documented. One method attempts to improve the oxygen utilization in continuous fermentation of single cells by recycling fermentation liquid. In this approach either air, enriched oxygen, or pure oxygen is used during fermentations. However, the only control applied in this method is the level of liquid in the fermentor. Another method focuses specifically on one type of microorganism (*Escherichia coli* bacteria), but methods of controlling the oxygen supply are not provided. Instead, the method teaches the regulation of the carbon source as a function of the oxygen uptake rate of the microorganism. Yet another method describes a method of increasing the oxygen transfer in a fermentation system by introducing oxygen in only one portion of the broth that is sent back to the fermentor. Still another method describes a method of utilizing high pressure in the fermentor to promote oxygen dissolution and low pressure to remove $CO_2$. Still other methods teach a method of enriching bubble fermentors with oxygen while using air bubbles to agitate the growth media and eliminate $CO_2$ accumulated in the media.

However, the foregoing methods each fail to provide a method to regulate the real-time oxygen supply in agitation and bubble fermentors to improve oxygen utilization and maximize the productivity of the fermentation, leading to favorable system economics.

Therefore, there remains a need for a method to optimize the use of pure oxygen in fermentation systems to maximize productivity, substrate yield, and oxygen utilization of the fermentation cell culture.

Aspects of the invention generally provide a method for controlling the addition of oxygen in fermentations to achieve a desired oxygen consumption and substrate yield in fermentations. In one aspect, the invention provides a method for regulating the addition of oxygen ($O_2$) to a fermentor during a fermentation process, comprising measuring real-time dissolved oxygen (DO) in a fermentation broth, measuring real-time $O_2$ concentration in the fermentor exhaust and providing the real-time DO measurement and real-time $O_2$ measurement to an adaptive controller configured to regulate $O_2$ flow into the fermentor responsive to the real-time DO measurement and real-time $O_2$ measurement.

In another aspect, the invention provides a method for regulating the addition of $O_2$ in a fermentor during a fermentation process, comprising measuring real-time dissolved oxygen (DO) in a fermentation broth, measuring real-time $O_2$ concentration in the fermentor exhaust, and providing the real-time DO measurement and real-time $O_2$ measurement to an adaptive controller, wherein the adaptive controller is configured to regulate incoming $O_2$ flow and agitation

speed in the fermentor responsive to the real-time DO measurement and real-time $O_2$ measurement.

In another aspect, the invention provides a method for regulating the addition of $O_2$ to a fermentor during a fermentation process, comprising measuring real-time dissolved oxygen (DO) in a fermentation broth, measuring real-time $O_2$ concentration in the fermentor exhaust, measuring an additional real-time parameter in the fermentation broth and providing the real-time DO measurement, real-time $O_2$ measurement, and additional real-time parameter measurement to an adaptive controller, wherein the adaptive controller is configured to regulate incoming $O_2$ flow and agitation speed in the fermentor responsive to the real-time DO measurement, real-time $O_2$ measurement and additional real-time parameter measurement.

In another aspect, the invention provides a method for regulating the addition of $O_2$ to a fermentor during a fermentation process, comprising measuring real-time dissolved oxygen (DO) in a fermentation broth, measuring real-time $O_2$ concentration in the fermentor exhaust, and providing the real-time DO measurement and real-time $O_2$ measurement to an adaptive controller configured to regulate $O_2$ and $N_2$ flow into the fermentor responsive to the real-time DO measurement and real-time $O_2$ measurement.

In another aspect, the invention provides a system for regulating addition of $O_2$ during a fermentation process, comprising a fermentor, a first measuring device configured for measuring real-time dissolved oxygen (DO) in a fermentation broth, a second measuring device configured for measuring real-time $O_2$ concentration in the fermentor exhaust, and an adaptive controller configured to regulate $O_2$ flow into the fermentor responsive to the real-time DO measurement and real-time $O_2$ measurement.

In another aspect, the invention provides a system for regulating addition of $O_2$ during a fermentation process, comprising a fermentor, a first measuring device configured for measuring real-time dissolved oxygen (DO) in a fermentation broth, a second measuring device configured for measuring real-time $O_2$ concentration in the fermentor exhaust, and an adaptive controller configured to regulate incoming $O_2$ flow and agitation speed in the fermentor responsive to the real-time DO measurement and real-time $O_2$ measurement.

In another aspect, the invention provides a system for regulating addition of $O_2$ during a fermentation process, comprising a fermentor, a first measuring device configured for measuring real-time dissolved oxygen (DO) in a fermentation broth, a second measuring device configured for measuring real-time $O_2$ concentration in the fermentor exhaust, a third measuring device configured for measuring an additional real-time parameter in the fermentation broth, and an adaptive controller configured to regulate incoming $O_2$ flow and agitation speed in the fermentor responsive to the real-time DO measurement, real-time $O_2$ measurement and additional real-time parameter measurement.

In another aspect, the invention provides a system for regulating addition of $O_2$ during a fermentation process, comprising a fermentor, a first measuring device configured for measuring real-time dissolved oxygen (DO) in a fermentation broth, a second measuring device configured for measuring real-time $O_2$ concentration in the fermentor exhaust, and an adaptive controller configured to regulate $O_2$ and $N_2$ flow into the fermentor responsive to the real-time DO measurement and real-time $O_2$ measurement.

For a further understanding of the nature and objects of the present invention, reference should be made to the following detailed description, taken in conjunction with the accompanying drawings, in which like elements are given the same or analogous reference numbers and wherein:

**Figure 1** is an embodiment of a process control system with two measurements and one manipulated variable in cascade form.

**Figure 2** is an embodiment of a process control system used to control the $O_2$ level in a fermentor which is mechanically agitated.

**Figure 3** is an embodiment of a process control block diagram system using a model-adaptive controller.

**Figure 4** is an embodiment of a process control system using an adaptive controller to control the $O_2$ level in a fermentor.

**Figure 5** is an embodiment of a process control system using an adaptive controller to control the $O_2$ level and agitation speed in a fermentor.

**Figure 6** is an embodiment of a process control system using an adaptive controller to control the $O_2$ level and agitation speed in a fermentor, including an additional sensing element for more real-time measurements.

**Figure 7** is an embodiment of a process control system using adaptive controllers to control the $O_2$ level and $N_2$ level in a bubble type fermentor.

The words and phrases used herein should be given their ordinary and customary meaning in the art by one skilled in the art unless otherwise further defined.

Aerobic fermentation is an important biochemical process typically conducted in a controlled reaction vessel. The environmental conditions in the vessel, such as gas exhaust content, temperature, pH, dissolved oxygen levels, and agitation speed rate need to be closely monitored and controlled to promote larger cell growth and product formation rates. The presence of pure oxygen in the fermentor promotes a high oxygen transfer rate to the microorganisms, enhancing growth and production formation. Embodiments of the present invention provide a system for manipulating the oxygen flow rate during an aerobic fermentation such that the productivity, yield and the oxygen transfer efficiency are maximized. The embodiments described herein offer methods and systems to accurately calculate the amount of pure oxygen to supply in an agitated fermentor based on real-time measurements of DO and $O_2$ exhaust concentrations, while maintaining desired agitation speed for desired pH levels or $CO_2$ removal. In bubble and airlift fermentors, the $N_2$ supply is controlled in addition to $O_2$ supply. Illustrative embodiments of the present invention include batch and fed-batch fermentations but other modes of operation are also contemplated.

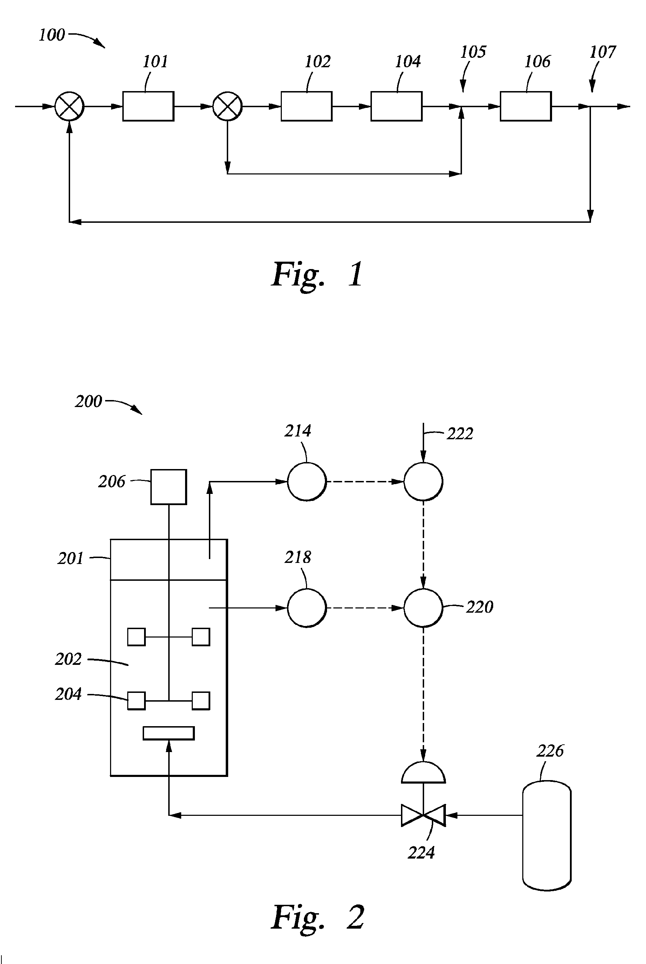

The embodiments of the invention include various process control systems to control the amount of pure oxygen supplied to a fermentor. **Figure 1** shows a process control system **100** using a cascade control. According to one embodiment, a cascade control is the combination of two or more controllers, where an output signal from one controller forms a setpoint of the other. A cascade control is used when there are two or more available measurements, but only one manipulated variable. In the process control system 100, a set of controllers **101** and **102** control a process that is subdivided into two separate processes. The output of process **104** is one process variable that is monitored, and **106** is another process whose output is the controlling variable. A process variable typically entails an intermediate procedure affecting a manipulated variable, or output. For example, in one embodiment, a process variable can involve the opening and closing of an $O_2$ valve connected to an $O_2$ source, thereby affecting a manipulated variable, the total $O_2$ flow rate. The process control system **100** allows the controller **101** to change the set point of the second controller **102**. The controller **102** measures one variable **105** and controls process **106** with the final controlling variable **107**.

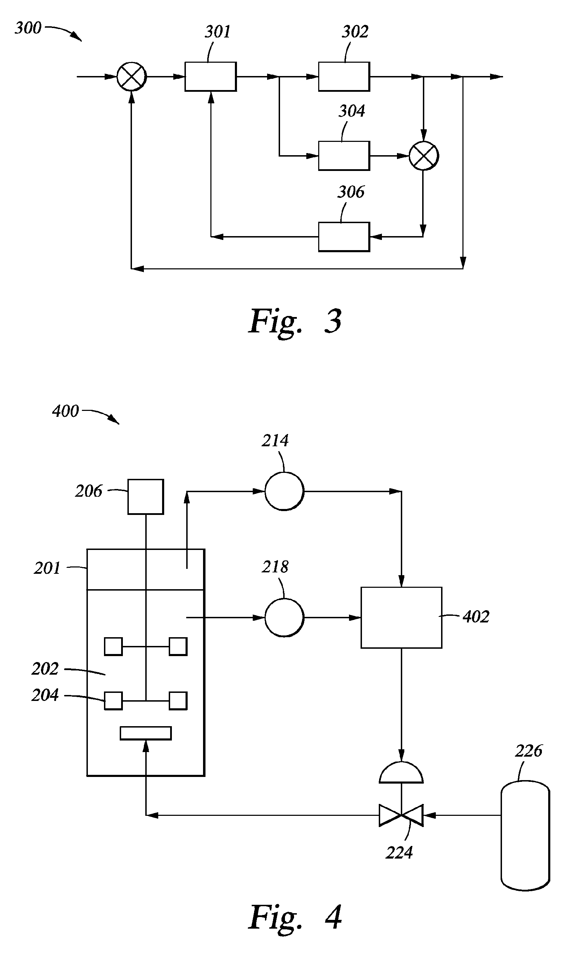

In one embodiment, the process control system **100** described above is applied to a fermentor. **Figure 2** is a diagram showing a process control system **200** using an embodiment of the cascade control system **100** to control the $O_2$ level in a fermentor **201**. The fermentor **201** includes a motor **206** connected to an impeller **204** which affects a desired agitation. In one embodiment, the agitation speed provided by the motor **206** and connected impeller **204** remains constant. The amount of $O_2$ exhaust in the headspace of the agitation fermentor **201** is measured by an oxygen sensor **214** and relayed to an $O_2$ controller **222**. The controller **222** is programmed to maintain a relatively low $O_2$ concentration level in the exhaust. The dissolved oxygen (DO) in a broth mixture **202** measured by the DO probe **218** and regulated by a DO controller **220**. An output signal from the $O_2$ controller **222** defines a setpoint for the DO controller **220**, which in turn regulates the oxygen flow rate through the opening of an $O_2$ valve **224** connected to an $O_2$ source **226**.

**Figure 3** shows a process control block diagram system **300**, according to another embodiment, in which a model-based adaptive controller is used. In one embodiment, the model-based adaptive controller may be applied to an agitated fermentor to control the supply of $O_2$, as will be described with respect to **Figure 4**, below. In this system, an adaptive controller **301** performs according to a specific model of a controlled process. The controller **301** is used to control measured model deviations from the desired process model. In one embodiment of the process control system **300**, only one manipulated variable applied to controller **302** is controlled by the adaptive controller **301**. An adaptation **306** is made after comparing the measurement of the process **302** to the output of the model of the controlled process **304**. The adaptation **306** affects the tuning parameters of the adaptive controller **301**, which in turn affects the manipulated variable applied to the process **302**.

As noted above, an embodiment of the process control system **300** described above can be applied to a mechanically agitated fermentor. **Figure 4** is a diagram showing a process control system **400** using an embodiment of the model-adaptive control system **300** to control the $O_2$ level in a mechanically agitated fermentor **201**. The adaptive controller **402** uses the real-time measurements from the DO probe **218** and the $O_2$ exhaust sensor **214**. The adaptive controller **402** compares these measurements to a specific process model. In one embodiment, the process model can be the behavior of the DO based on the gas supply. After the comparison with the process model, the adaptive controller **402** regulates the oxygen flow rate through the opening of an $O_2$ valve **224** connected to an $O_2$ source **226**.

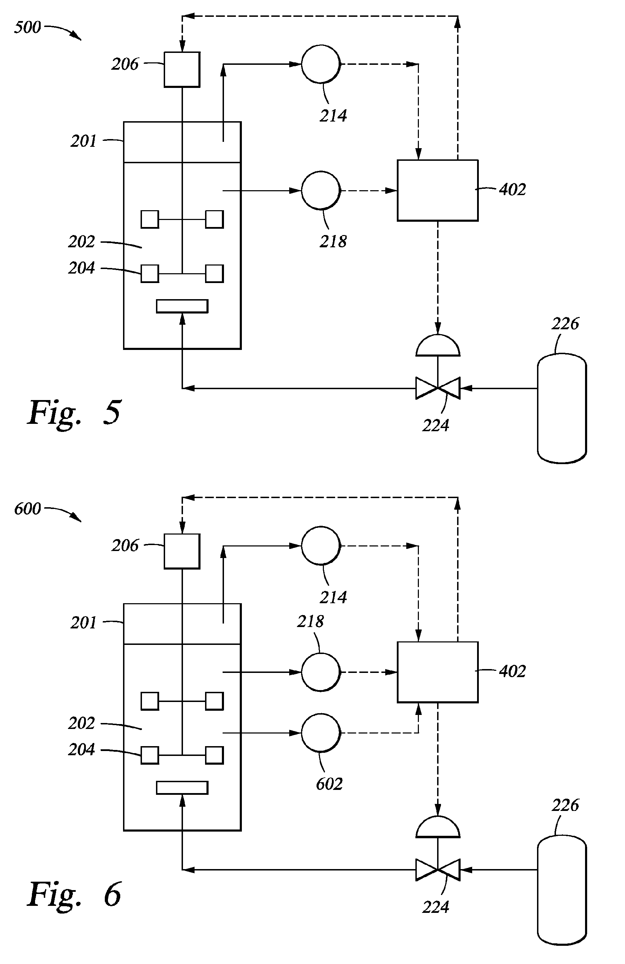

The agitation speed of a rotor inside a fermentor can affect the amount of DO and $O_2$ exhaust during a fermentation. Accordingly, in another embodiment, the agitation speed of the rotor is controlled. **Figure 5** exhibits an embodiment of a process control system **500** in which the model-adaptive control system **300** controls the $O_2$ level and agitation speed in a mechanically agitated fermentor **201**. The adaptive controller **402** uses the real-time measurements from the DO probe **218** and the $O_2$ exhaust sensor **214**. The adaptive controller **402** compares these measurements to a specific process model, and regulates the oxygen flow rate through the opening of an $O_2$ valve **224** connected to an $O_2$ source **226**. The adaptive controller **402** also regulates the agitation speed of the motor **206** connected to the agitator **204** in the agitation fermentor **201**.

Additional variables such as pH, cell density, and cell product formation can help determine the optimum amount

of $O_2$ to be supplied during a fermentation. **Figure 6** exhibits a process control system **600** using another embodiment of the model-adaptive control system **300** to control the $O_2$ level in a fermentor **201**. The adaptive controller **402** uses the real-time measurements from the DO probe **218**, the $O_2$ exhaust sensor **214**, and an additional sensing element **602**. The additional sensing element can measure variables in the fermentation broth **202** related the cell mass growth (pH, optical density) or cell products during fermentation. The adaptive controller **402** compares these measurements to a specific process model, and regulates the oxygen flow rate through the opening of an $O_2$ valve **224** connected to an $O_2$ source **226**. The adaptive controller **402** also regulates the agitation speed of the motor **206** connected to the agitator **204** in the agitation fermentor **201**.

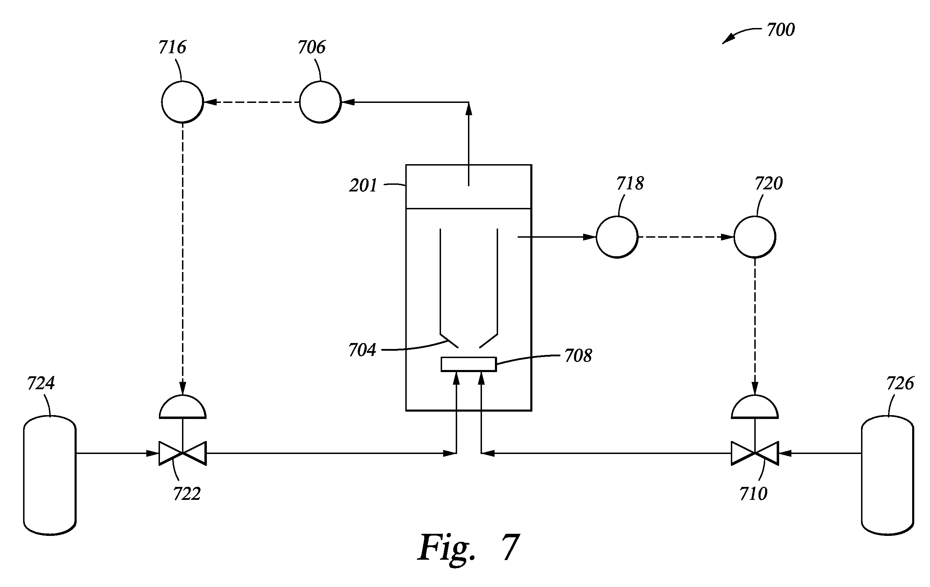

Addition of pure oxygen to bubble type or airlifted fermentors may require the removal of excess $CO_2$. To accomplish this, an additional injection of $N_2$ may be utilized to remove $CO_2$ and provide extra mixing, according to one embodiment. Therefore, the manipulated variables in the bubble fermentor are the $O_2$ and $N_2$ flow rates. **Figure 7** is an embodiment showing a process control system **700** having a bubble fermentor **701**, in which bubbles generated at a gas injection system **708** provide the agitation. The injector **708** can consist of a gas distribution plate of varying diameter located at the lower section of the bioreactor. The injector **708** is directly connected to the gas sources. In this embodiment, a controller **716** regulates the $O_2$ flow rates through the opening of an $O_2$ valve **722** connected to an $O_2$ source **724**. Another controller **720** regulates the $N_2$ flow rates through the opening of a $N_2$ valve **710** connected to a $N_2$ source **726**. The controlled variables in this embodiment are DO measured by a DO sensor **718**, and $O_2$ level in the fermentor measured by an $O_2$ exhaust sensor **706**. An inner draft tube **704** prevents the coalescing of bubbles and promotes efficient mixing in the fermentor. An airlift reactor is another possible embodiment similar in design to this figure without the inner draft tube **704**.

The following example is presented for a further understanding of the nature and objects of the present invention. The example is illustrative only and other embodiments of the integrated processes and apparatus may be employed without departing from the true scope of the invention.

This example describes one model that can be used to control oxygen flow into a fermentor during an aerobic fermentation. The model can consist of following equations, which reflect the most important interactions during the fermentation process when a gas flow rate into the fermentor is controlled by an adaptive controller:

$$\mu = \frac{\mu_m S}{(K_s + S)} \qquad (1)$$

$$\mu_o = \frac{\mu_{om} O_2}{(K_o + O_2)} \qquad (2)$$

$$\frac{dX}{dt} = (\mu * \mu_o) X \qquad (3)$$

$$\frac{dS}{dt} = -\mu \ X / Y_{XS} \qquad (4)$$

$$\frac{dP}{dt} = \mu X / Y_{XP} \qquad (5)$$

$$\frac{dO_2}{dt} = kla(O_2^* - O_2) - \mu_o X / Y_{xo} = OTR - OCR \qquad (6)$$

$$F_{o,exit} = F_i - OTR \tag{7}$$

$$OTR = kla * 1.15 * F_i (O_2^* - O_2) \tag{8}$$

$$M(k) = M(k-1) + b_o E(k-1) + b_1 ((E(k) - E(k-1))) \tag{9}$$

<u>Nomenclature:</u>

| | |
|---|---|
| $\mu$ | Substrate growth rate |
| $\mu_m$ | Substrate specific growth rate |
| S | Substrate concentration |
| $K_s$ | Substrate inhibition constant |
| $\mu_o$ | Oxygen growth rate |
| $\mu_{om}$ | Oxygen specific growth rate |
| $O_2$ | Oxygen concentration |
| X | Cell mass concentration |
| $Y_{xs}$ | Cell mass to substrate yield |
| P | Product concentration |
| $Y_{xp}$ | Cell mass to product yield |
| kla | Mass transfer coefficient |
| $O_2^*$ | Equilibrium oxygen concentration in the broth |
| $Y_{xo}$ | Cell mass to oxygen yield |
| OTR | Oxygen transfer rate |
| OCR | Oxygen consumption rate |
| $F_{o,exit}$ | Oxygen flow exiting the fermentor |
| $F_i$ | Oxygen flow entering the fermentor |
| M | Manipulating variable |
| E | Error between setpoint and $O_2$ in media |
| $b_0$ | Controller tuning parameter |
| $b_1$ | Controller tuning parameter |
| k | Time interval |

Equation **1** represents a typical microorganism growth rate represented by the Monod Equation. The microorganism growth rate can be influenced by $O_2$ as a substrate in a fermentor, and Equation **1** can be modified to Equation **2** to include the addition of $O_2$. The overall cell mass concentration can be represented as the multiplicative contribution of the substrate and the oxygen concentrations. Equation **3** represents the change in cell mass concentration as a function of the substrate growth rate and oxygen growth rate. Equation **4** represents the substrate consumption and Equation **5** represents the product formation during a fermentation. Both the substrate consumption and product formation rates are limited by the corresponding yields. Equation **6** reflects the overall $O_2$ available in the media, which is a function of the oxygen transferred from the gas phase to the media (OTR) and the oxygen consumed by the microorganisms (OCR).

There is a continuous supply and removal of gas from the fermentor in this model. Using a material balance, the amount of gas exiting the fermentor is calculated as the difference of the gas supply and the oxygen transferred to the media (OTR) as shown in Equation **7**, the OTR being calculated in Equation **8**. In reality, the mass transfer coefficient, kla, is a function of the inlet gas flow rate as it changes the size of gas bubbles. The mass transfer coefficient can also change with time due to physical properties of the media during the fermentation, and an example of a time varying kla is given by Equation **10**:

$$kla = 1e\text{-}06*t^2\text{-}0.0001*t+0.0038; \qquad (10)$$

In order to perform a control experiment, a control algorithm is needed. A controller that can be used in this model is known as a proportional plus integral controller (PI). Equation **9** represents a controller in discrete form, in which M is the manipulating variable, in this case the gas inlet flow, $F_i$, and E is the error between the set point and the controlled variable, the $O_2$ in the media. The constants $b_0$ and $b_1$ are the controller tuning parameters, and k represents the time interval. The parameters in Equation **9** are constant parameters that are satisfactory for processes that do not change significantly with time. However, as shown in Equation **10,** fermentation processes can change significantly with time. Therefore, the controller parameters, $b_0$ and $b_1$, are not necessarily kept constant. An example of a simple adaptive controller is given by Equation **11,** which shows that the controller parameter changes as the $O_2$ measurement changes:

$$bo = -21.55*O_2+2.1664 \qquad (11)$$

Processes and apparatus for practicing the present invention have been described. It will be understood and readily apparent to the skilled artisan that many changes and modifications may be made to the above-described embodiments without departing from the spirit and the scope of the present invention. The foregoing is illustrative only and other embodiments of the integrated processes and apparatus may be employed without departing from the true scope of the invention defined in the following claims.

**Claims**

1. A method for regulating the addition of oxygen ($O_2$) to a fermentor during a fermentation process, comprising:

    a) measuring real-time dissolved oxygen (DO) in a fermentation broth;
    b) measuring real-time $O_2$ concentration in fermentor exhaust of the fermentor; and
    c) providing the real-time DO measurement and real-time $O_2$ measurement to an adaptive controller configured to regulate $O_2$ flow into the fermentor responsive to the real-time DO measurement and real-time $O_2$ measurement.

2. The method of claim 1, wherein the fermentor is mechanically agitated fermentor.

3. The method of claim 1, further comprising a probe in the fermentation broth configured to measure the real-time DO.

4. The method of claim 1, wherein the real-time $O_2$ concentration in the fermentor exhaust is measured by a sensor in a headspace of the fermentor.

5. The method of claim 1, wherein the adaptive controller regulates $O_2$ flow into the fermentor by acting on an $O_2$ control valve connected to an $O_2$ source.

6. The method of claim 1, wherein the adaptive controller regulates $O_2$ flow into the fermentor by implementing a model which defines a desired process.

7. A method for regulating the addition of $O_2$ in a fermentor during a fermentation process, comprising:

    a) measuring real-time dissolved oxygen (DO) in a fermentation broth;
    b) measuring real-time $O_2$ concentration in fermentor exhaust of the fermentor; and
    c) providing the real-time DO measurement and real-time $O_2$ measurement to an adaptive controller, wherein the adaptive controller is configured to regulate incoming $O_2$ flow and agitation speed in the fermentor responsive to the real-time DO measurement and real-time $O_2$ measurement.

8. The method of claim 7, wherein the fermentor is an mechanically agitated fermentor.

**9.** The method of claim 7, further comprising a probe in the fermentation broth configured to measure the real-time DO.

**10.** The method of claim 7, wherein the real-time $O_2$ concentration in the fermentor exhaust is measured by a sensor in a headspace of the fermentor.

**11.** The method of claim 7, wherein the adaptive controller regulates $O_2$ flow into the fermentor by acting on an $O_2$ control valve connected to an $O_2$ source.

**12.** The method of claim 7, wherein the adaptive controller regulates the agitation speed in the fermentor by acting on a motor connected to an agitator in the fermentation broth.

**13.** The method of claim 7, wherein the adaptive controller regulates $O_2$ flow into the fermentor by implementing a model which defines a desired process.

**14.** A method for regulating the addition of $O_2$ to a fermentor during a fermentation process, comprising:

a) measuring real-time dissolved oxygen (DO) in a fermentation broth;
b) measuring real-time $O_2$ concentration in fermentor exhaust of the fermentor;
c) measuring an additional real-time parameter in the fermentation broth; and
d) providing the real-time DO measurement, real-time $O_2$ measurement, and additional real-time parameter measurement to an adaptive controller, wherein the adaptive controller is configured to regulate incoming $O_2$ flow and agitation speed in the fermentor responsive to the real-time DO measurement, real-time $O_2$ measurement and additional real-time parameter measurement.

**15.** The method of claim 14, wherein the fermentor is a mechanically agitated fermentor.

**16.** The method of claim 14, further comprising a probe in the fermentation broth configured to measure the real-time DO.

**17.** The method of claim 14, wherein the real-time $O_2$ concentration in the fermentor exhaust is measured by a sensor in a headspace of the fermentor.

**18.** The method of claim 14, wherein the additional parameter measured can be cell density in the fermentation broth, pH of the fermentation broth, temperature of the broth, quantity of cellular products in the fermentation broth, or carbon dioxide ($CO_2$) concentration in the fermentor.

**19.** The method of claim 14, wherein the adaptive controller regulates $O_2$ flow into the fermentor by acting on an $O_2$ control valve connected to an $O_2$ source.

**20.** The method of claim 14, wherein the adaptive controller regulates the agitation speed in the fermentor by acting on a motor connected to an agitator in the fermentation broth.

**21.** The method of claim 14, wherein the adaptive controller regulates $O_2$ flow into the fermentor by implementing a model which defines a desired process.

**22.** A method for regulating the addition of $O_2$ to a fermentor during a fermentation process, comprising

a) measuring real-time dissolved oxygen (DO) in a fermentation broth;
b) measuring real-time $O_2$ concentration in fermentor exhaust of the fermentor; and
c) providing the real-time DO measurement and real-time $O_2$ measurement to an adaptive controller configured to regulate $O_2$ and $N_2$ flow into the fermentor responsive to the real-time DO measurement and real-time $O_2$ measurement.

**23.** The method of claim 22, wherein the fermentor is a bubble fermentor.

**24.** The method of claim 22, wherein the fermentor is an airlift fermentor.

**25.** The method of claim 22, further comprising a probe in the fermentation broth configured to measure the real-time DO.

**26.** The method of claim 22, wherein the real-time $O_2$ concentration in the fermentor exhaust is measured by a sensor in a headspace of the fermentor.

**27.** The method of claim 22, wherein the adaptive controller regulates $O_2$ flow into the fermentor by acting on an $O_2$ control valve connected to an $O_2$ source.

**28.** The method of claim 22, wherein the adaptive controller regulates $O_2$ flow into the fermentor by implementing a model which defines a desired process.

**29.** A system for regulating addition of $O_2$ during a fermentation process, comprising:

a) a fermentor;
b) a first measuring device configured for measuring real-time dissolved oxygen (DO) in a fermentation broth;
c) a second measuring device configured for measuring real-time $O_2$ concentration in fermentor exhaust of the fermentor; and
d) an adaptive controller configured to regulate $O_2$ flow into the fermentor responsive to the real-time DO measurement and real-time $O_2$ measurement.

**30.** A system for regulating addition of $O_2$ during a fermentation process, comprising:

a) a fermentor;
b) a first measuring device configured for measuring real-time dissolved oxygen (DO) in a fermentation broth;
c) a second measuring device configured for measuring real-time $O_2$ concentration in fermentor exhaust of the fermentor; and
d) an adaptive controller configured to regulate incoming $O_2$ flow and agitation speed in the fermentor responsive to the real-time DO measurement and real-time $O_2$ measurement.

**32.** A system for regulating addition of $O_2$ during a fermentation process, comprising:

a) a fermentor;
b) a first measuring device configured for measuring real-time dissolved oxygen (DO) in a fermentation broth;
c) a second measuring device configured for measuring real-time $O_2$ concentration in fermentor exhaust of the fermentor;
d) a third measuring device configured for measuring an additional real-time parameter in the fermentation broth; and
e) an adaptive controller configured to regulate incoming $O_2$ flow and agitation speed in the fermentor responsive to the real-time DO measurement, real-time $O_2$ measurement and additional real-time parameter measurement.

**33.** A system for regulating addition of $O_2$ during a fermentation process, comprising:

a) a fermentor;
b) a first measuring device configured for measuring real-time dissolved oxygen (DO) in a fermentation broth;
c) a second measuring device configured for measuring real-time $O_2$ concentration in fermentor exhaust of the fermentor; and
d) an adaptive controller configured to regulate $O_2$ and $N_2$ flow into the fermentor responsive to the real-time DO measurement and real-time $O_2$ measurement.

*100*

*101*    *102*    *104*    *105*    *106*    *107*

## Fig. 1

*200*

*206*

*201*

*202*

*204*

*214*    *222*

*218*

*220*

*226*

*224*

## Fig. 2

300

301    302

304

306

**Fig. 3**

400

206

214

218

201

202

204

402

226

224

**Fig. 4**

500

206

201

202

204

214

218

402

226

224

## Fig. 5

600

206

201

202

204

214

218

402

602

226

224

## Fig. 6

Fig. 7

European Patent
Office

**EUROPEAN SEARCH REPORT**

Application Number

EP 06 11 4858

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 029 101 A (YOSHIDA ET AL) 22 February 2000 (2000-02-22) * column 1, lines 15-24,60-67 * * column 3, lines 4-11 * * column 5, lines 1-19,29-36 * * column 6, lines 1-8,21-26 * * column 7, lines 26-39 * ----- | 1-21,29, 30,32 | INV. C12M1/04 C12M1/06 C12M1/36 |
| X | US 3 926 738 A (NYIRI ET AL) 16 December 1975 (1975-12-16) * column 1, lines 6-9,38-46 * * column 3, lines 12-15 * * column 8, lines 30-34 * ----- | 1-6, 14-19, 21,29 | |
| X | US 5 126 238 A (GEBHARD ET AL) 30 June 1992 (1992-06-30) * column 1, lines 26-34 * * column 3, lines 21-31 * * column 4, lines 4-9 * * column 5, lines 44-64 * * column 13, lines 60-65 * ----- | 1-6, 14-19, 21,29 | |
| X | GB 1 457 571 A (GENERAL ELECTRIC CO) 8 December 1976 (1976-12-08) * page 1, lines 41-82 * * claims; figure * ----- | 1-4, 14-17 | TECHNICAL FIELDS SEARCHED (IPC) C12M B01F |
| A | US 5 985 652 A (CHENG ET AL) 16 November 1999 (1999-11-16) * column 2 - column 3 * ----- | 1,7,14, 22,29, 30,32,33 | |

-/--

The present search report has been drawn up for all claims

| Place of search | Date of completion of the search | Examiner |
|---|---|---|
| Berlin | 25 September 2006 | Böhm, Ingo |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**European Patent Office**

**EUROPEAN SEARCH REPORT**

Application Number

EP 06 11 4858

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2004/044183 A (AKZO NOBEL N.V; LIN, WENGLONG, R; MISTRY, FIROZ, R; NARAYANASWAMY, ARU) 27 May 2004 (2004-05-27) * page 9, paragraph 1 * * page 11, paragraph 1 * * page 13, paragraph 2 * * claims * ----- | 1,7,14, 22,29, 30,32,33 | |

TECHNICAL FIELDS
SEARCHED      (IPC)

The present search report has been drawn up for all claims

| Place of search | Date of completion of the search | Examiner |
|---|---|---|
| Berlin | 25 September 2006 | Böhm, Ingo |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 06 11 4858

This annex lists the patent family members relating to the patent documents cited in the above-mentioned European search report.
The members are as contained in the European Patent Office EDP file on
The European Patent Office is in no way liable for these particulars which are merely given for the purpose of information.

25-09-2006

| Patent document cited in search report | | Publication date | Patent family member(s) | | Publication date |
|---|---|---|---|---|---|
| US 6029101 | A | 22-02-2000 | NONE | | |
| US 3926738 | A | 16-12-1975 | CH | 558017 A | 15-01-1975 |
| | | | DE | 2323416 A1 | 29-11-1973 |
| | | | FR | 2184035 A1 | 21-12-1973 |
| | | | GB | 1434613 A | 05-05-1976 |
| | | | JP | 49050989 A | 17-05-1974 |
| | | | JP | 56051760 B | 08-12-1981 |
| US 5126238 | A | 30-06-1992 | US | 5656421 A | 12-08-1997 |
| GB 1457571 | A | 08-12-1976 | NONE | | |
| US 5985652 | A | 16-11-1999 | BR | 9704700 A | 29-12-1998 |
| | | | DE | 69709276 D1 | 31-01-2002 |
| | | | DE | 69709276 T2 | 08-08-2002 |
| | | | EP | 0829534 A2 | 18-03-1998 |
| | | | ES | 2165553 T3 | 16-03-2002 |
| | | | US | 5798254 A | 25-08-1998 |
| WO 2004044183 | A | 27-05-2004 | AU | 2003298649 A1 | 03-06-2004 |
| | | | US | 2005202525 A1 | 15-09-2005 |
| | | | US | 2004091954 A1 | 13-05-2004 |

EPO FORM P0459

For more details about this annex : see Official Journal of the European Patent Office, No. 12/82

**EP 1 728 853 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 60686730 B